(19) Europäisches Patentamt
European Patent Office
Office européen des brevets

(11) Numéro de publication : **0 009 632**
**B1**

(12) **FASCICULE DE BREVET EUROPÉEN**

(45) Date de publication du fascicule du brevet : **24.02.82**

(21) Numéro de dépôt : **79103231.1**

(22) Date de dépôt : **31.08.79**

(51) Int. Cl.³ : **C 07 C 69/587**, A 61 K 7/46, A 23 L 1/226, A 23 L 1/235, A 24 B 3/12, A 24 B 15/18

(54) **Esters aliphatiques polyinsaturés, procédé pour la préparation de ces esters et leur utilisation en tant qu'ingrédients aromatisants et parfumants.**

(30) Priorité : **31.08.78 CH 9194/78**

(43) Date de publication de la demande : **16.04.80 (Bulletin 80/08)**

(45) Mention de la délivrance du brevet : **24.02.82 Bulletin 82/08**

(84) Etats contractants désignés : **CH DE FR GB IT NL**

(56) Documents cités :
**CH - A - 544 511**
**CH - A - 544 803**
**CH - A - 557 645**
**CH - A - 561 271**
**DE - A1 - 2 433 408**
**GB - A - 1 379 038**
**US - A - 3 953 377**
**US - A - 4 094 823**

(73) Titulaire : **FIRMENICH SA**
**Case postale 239**
**CH-1211 Genève 8 (CH)**

(72) Inventeur : **Näf, Ferdinand**
**11, ch. des Crêts de Champel**
**CH-1206 Geneve (CH)**
Inventeur : **Giersch, Wolfgang**
**353, Route de Bernex**
**CH-1233 Bernex/Ge (CH)**
Inventeur : **Ohloff, Günther**
**13, ch. de la Chapelle**
**CH-1233 Bernex/Ge (CH)**

(74) Mandataire : **Schmied-Kowarzik, Volker, Dr. et al**
**Patentanwälte Dipl.-Ing. P. Wirth Dr. V. Schmied-Kowarzik Dipl.-Ing. G. Dannenberg, Dr. P. Weinhold Dr. D. Gudel, Dipl.-Ing. S. Schubert**
**Siegfriedstrasse 8 D-8000 München 40 (DE)**

**EP 0 009 632 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Imprimerie Jouve, 18, rue St-Denis, 75001 Paris, France

Esters aliphatiques polyinsaturés, procédé pour la préparation de ces esters et leur utilisation en tant qu'ingrédients aromatisants et parfumants

La présente invention se rapporte au domaine des arômes et à celui de la parfumerie. Elle a trait en particulier à l'utilisation d'esters aliphatiques polyinsaturés de formule

$$CH_2{=}CH{-}CH_2{-}\overset{4}{C}H{=}CH{-}CH_2{-}CH_2{-}\overset{\overset{O}{\|}}{C}{-}OR \qquad (I)$$

dans laquelle le symbole R représente un radical alkyle inférieur, linéaire ou ramifié, contenant 1 à 3 atomes de carbone et dans laquelle la double liaison à la position 4 possède la configuration cis, en tant qu'ingrédients parfumants pour la préparation de parfums et produits parfumés, et en tant qu'agents aromatisants pour l'aromatisation d'aliments, de boissons, de préparations pharmaceutiques et du tabac. Le radical alkyle R est méthyle, éthyle, propyle ou isopropyle.

La présente invention se rapporte en outre à une composition parfumante ou aromatisante caractérisée en ce qu'elle contient en tant qu'ingrédient actif un ester aliphatique polyinsaturé de formule (I).

L'invention a également trait à un aliment ou une boisson contenant en tant qu'ingrédient aromatisant un ester aliphatique polyinsaturé de formule (I).

Les composés de formule (I) sont des composés nouveaux.

L'état de la technique fait état de l'utilisation de certains esters aliphatiques insaturés en tant qu'agents parfumants et aromatisants. Les brevets suisses no. 557 645 et 561 271 notamment décrivent l'utilisation, en tant qu'agents aromatisants, respectivement parfumants, des composés carbonylés insaturés de formule

$$R{-}\overset{\delta}{C}H{=}\overset{\gamma}{C}H{-}\overset{\beta}{C}H_n\,{\cdots\cdots}\,\overset{\alpha}{C}H_n{-}\overset{\overset{O}{\|}}{C}{-}O{-}R^1$$

possédant une double liaison de configuration cis- en position $\gamma$, $\delta$, ainsi qu'une simple ou double liaison, de configuration trans-, en position $\alpha$, $\beta$, et dans laquelle l'indice n vaut 1 ou 2, le symbole R représente un reste alkyle linéaire contenant 3, 8, 9, 10 ou 11 atomes de carbonne et $R^1$ un reste méthyle ou éthyle.

Les composés cités ci-dessus sont utilisés afin de renforcer l'effet naturel de nombreuses compositions parfumantes et présentent des notes de caractère fruité, légèrement vertes, grasses, rappelant parfois celles de la poire.

Il a été maintenant découvert que les composés de la présente invention définis par la formule (I) possèdent des propriétés organoleptiques intéressantes ou inattendues, propriétés qui se distinguent de celles des composés décrits dans les antériorités citées ci-dessus. Tout en ayant des propriétés organoleptiques de type fruité, ils se distinguent des composés de l'état de la technique par le fait de posséder une note fruitée rappelant typiquement le goût et l'arôme développé par l'ananas, le fruit de la passion et même l'orange. Leur utilisation, par conséquent, permet de reproduire plus fidèlement le goût propre de ces fruits, plus spécialement de leurs jus, en renforçant leur caractère gustatif. Les composés de l'invention trouvent une utilisation fort appréciée dans l'aromatisation d'aliments et de boissons de nature diverse, mais surtout dans l'aromatisation de produits laitiers, des yogourts, par exemple, de jus de fruits et des sirops. Ils peuvent également servir à l'aromatisation de produits destinés à la boulangerie ou la pâtisserie, ou des confitures et des bonbons par exemple, ou des chewing-gums dans lesquels la note fruitée est désirée.

Les proportions dans lesquelles les composés de formule (I) développent des effets aromatisants intéressants sont comprises dans une gamme étendue de valeurs. De préférence, ils sont utilisés à des concentrations comprises entre environ 0,5 et 50 ppm (parties par million) en poids par rapport au poids total de l'aliment dans lequel ils sont incorporés. Bien entendu, les limites indiquées ci-dessus ne sauraient être interprétées de façon restrictive, l'utilisateur pouvant déterminer la teneur exacte désirée suivant la nature des produits soumit à l'aromatisation et le goût spécifique souhaité.

Les composés de l'invention peuvent être utilisés à l'état isolé ou, de préférence, sous forme d'une composition en mélange avec d'autres ingrédients aromatisants. Ils peuvent être incorporés au cours de n'importe quel stade de fabrication de l'aliment ou de la boisson choisis, et de préférence ils sont ajoutés sous forme de solution dans l'un des solvants comestibles usuels, tel l'alcool éthylique, le dipropylène-glycol ou la triacétine.

Parmi les composés de formule (I), l'octa-cis 4,7-diénoate d'éthyle représente le composé préférentiel pour toute utilisation en tant qu'ingrédient aromatisant conformément à l'invention.

Lorsque les composés de formule (I) sont utilisés en tant qu'ingrédients parfumants, ils développent

des notes olfactives fraîches, fleuries et fruitées, voire vertes.

Les esters de l'invention sont préparés conformément à un procédé original à partir de cycloocta-diène. Ce procédé, qui constitue également un des objets de la présente invention, est caractérisé en ce qu'on

a) époxyde sélectivement le cycloocta-1,5-diène pour fournir le 1,2-époxy-cyclooct-5-ène,

b) oxyde le composé ainsi obtenu pour donner le 1-oxo-2-hydroxy-cyclooct-5-ène,

c) traite ledit céto-alcool avec du tétraacétate de plomb en milieu éthanolique pour fournir le 7-formyl-hept-cis-4-ène-1-oate d'éthyle,

d) réduit ce dernier à l'aide de borhydrure de sodium,

e) estérifie le 8-hydroxy-oct-cis-4-ène-1-oate d'éthyle résultant pour fournir le 8-acétoxy-oct-cis-4-ène-1-oate d'éthyle et

f) soumet ledit ester à pyrolyse pour fournir l'octa-cis-4,7-diène-1-oate d'éthyle, et, le cas échéant, trans-estérifie ledit ester.

Le procédé de l'invention est illustré par le schéma réactionnel que voici :

Schéma

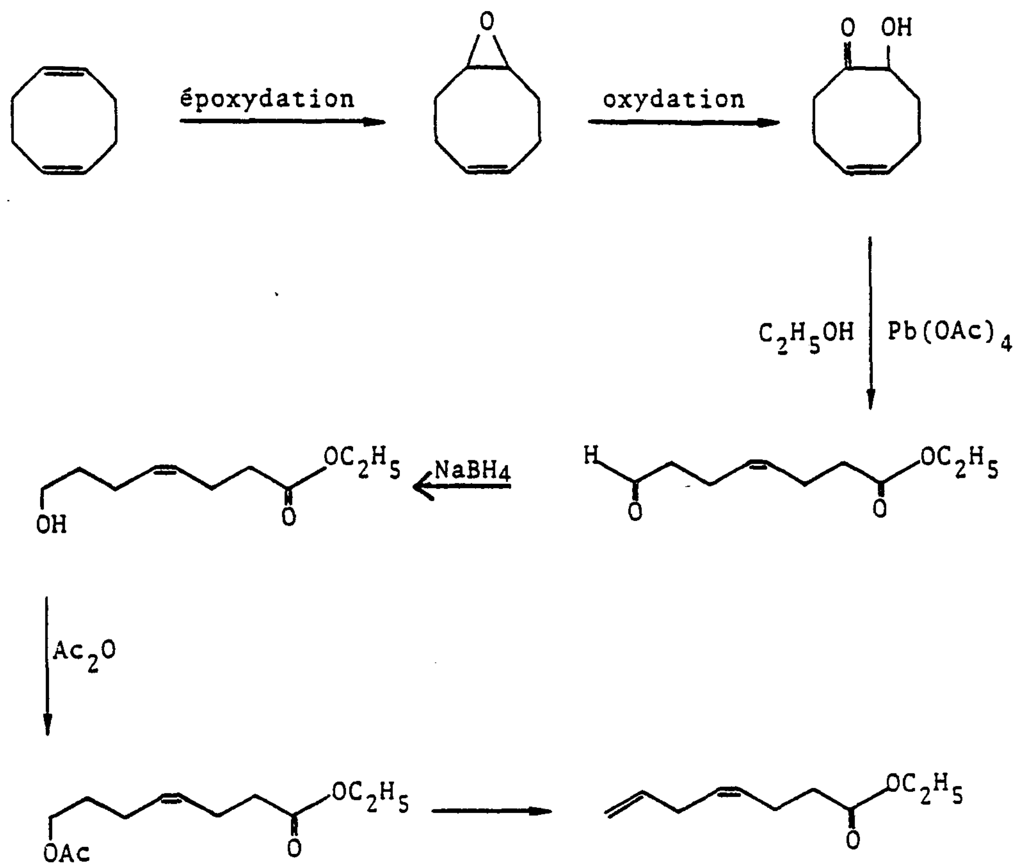


L'invention est illustrée de manière plus détaillée par les exemples suivants (températures en degrés centigrades).

## Exemple 1

Octa-cis 4,7-diène-1-oate-d'hétyle

1. 20 g d'acide m-chloroperbenzoïque (titre env. 85 %) dans 1 l de dichlorométhane ont été ajoutés goutte à goutte à une température d'environ 5° à une solution de 45 g de cyclo-octa-1,5-diène dans 500 ml de dichlorométhane. Le mélange réactionnel a été ensuite maintenu 18 h à température ambiante, puis après filtration, lavé avec une solution aqueuse de sulfite de sodium, du carbonate de sodium et enfin avec de l'eau jusqu'à neutralité. Par évaporation des parties volatiles et distillation on a obtenu 32 g d'une fraction ayant Eb. 41°/0,01 Torr.

2. La substance ainsi obtenue a été dissoute dans 150 ml de diméthylsulfoxyde, puis à travers la solution formée chauffée à 110° on a introduit pendant 27 h un courant d'air. Le déroulement de la réaction a été suivi par des analyses successives au moyen de chromatographie en phase gazeuse. Après refroidissement le mélange de réaction a été versé dans de l'eau glacée et extrait à l'éther. Les extraits organiques combinés ont été traités conformément aux méthodes habituelles de lavage, séchage et évaropation, et enfin distillés.

On a ainsi obtenu 21 g de 1-oxo-2-hydroxy-cyclooct-5-ène ayant Eb. 63-70°/0,05 Torr.

IR : 3 400, 1 700, 835 $cm^{-1}$ ;

RMN : 1,2-2,9 (8H, m) ; 3,4 (1H) ; 4,33 (1H, m) ; 5,65 (1H, m) δ ppm ;

SM : $M^+$ = 140 (3) ; m/e : 122 (16), 111 (18), 96 (60), 84 (56), 68 (95), 67 (100), 54 (85), 39 (68), 27 (46).

3. 80 g de tétraacétate de plomb ont été ajoutés à température ambiante et par petites portions à une solution maintenue sous agitation de 21 g de l'hydroxy-cétone obtenue ci-dessus dans 500 ml d'un mélange 3 : 7 d'éthanol et toluène. L'addition a duré environ 15 min et la température du mélange a augmenté jusqu'à environ 35°. Après avoir été maintenu au repos pendant 1 h 30 min, le mélange de réaction a été filtré, puis le filtrat clair a été lavé avec une solution aqueuse de bisulfite de sodium, de carbonate de sodium et avec de l'eau salée puis, par évaporation et distillation, on a obtenu à Eb. 120°/0,1 Torr, 12,2 g de 7-formyl-hept-cis 4-ène-1-oate d'éthyle ayant une pureté d'environ 85 %.

IR : 2 740, 1 730, 855 $cm^{-1}$ ;

RMN : 1,27 (3H, t, J = 7 Hz) ; 1,5-2,7 (8H, m) ; 4,17 (2H, q, J = 7 Hz) ; 5,4 (1H, m) ; 9,77 (1H, m) δ ppm ;

SM : $M^+$ = 184 (0) ; m/e : 141 (40), 110 (15), 95 (22), 81 (33), 67 (100), 55 (49), 41 (69), 29 (67).

4. Un mélange constitué par 7 g de l'oxo-ester obtenu sous chiffre 3 ci-dessus et 2 g de borhydrure de sodium dans 50 ml d'éthanol a été maintenu sous agitation à température ambiante pendant 2 h. Le mélange de réaction a été ensuite versé sur de la glace, acidifié et extrait à l'éther. Par les traitements habituels de lavage, séchage et évaporation, suivis de distillation au moyen d'un appareil à bulles on a obtenu 5,2 g de 8-hydroxy-oct-cis-4-ène-1-oate d'éthyle ayant Eb. 140° (température de bain)/0,01 Torr.

IR : 3 450, 1 740 et 855 $cm^{-1}$ ;

RMN : 1,23 (t, J = 7 Hz, 3H), 1,65 (m, 2H) ; 2,02 (1H) ; 1,9-2,5 (m, 6H) ; 3,67 (t, J = 6 Hz, 2H) ; 4,13 (q, J = 7 Hz, 2H) ; 5,4 (m, 2H) δ ppm ;

SM : $M^+$ = 186 (0) ; m/e : 156 (59), 140 (31), 123 (26), 110 (47), 97 (54), 81 (92), 67 (100), 55 (58), 41 (76), 29 (83).

5. Un mélange constitué par 4 g de l'ester hydroxylé obtenu ci-dessus, 10 ml d'anhydride acétique et 10 ml de pyridine a été maintenu sous agitation à température ambiante pendant 2 h. Le mélange brut a été ensuite soumis directement à pyrolyse à une température de 450° dans un courant d'azote. Le pyrolysat a été ensuite dissout dans de l'éther de pétrole et la solution ainsi obtenue soumise aux traitements usuels de lavage avec une solution aqueuse d'acide chlorhydrique dilué, de bicarbonate de sodium et avec de l'eau. L'ester désiré obtenu a été purifié au moyen d'une chromatographie sur colonne remplie de silice pour donner 480 mg d'un produit présentant les caractères analytiques suivants ; Eb. (four à boules) : 72-78°/0,05 Torr.

IR : 3 080, 1 735, 1 640, 920 et 750 $cm^{-1}$ ;

RMN : 1,25 (3H, t, J = 7 Hz) ; 2,38 (m, 4H) ; 2,82 (2H, t, J = 5 Hz) ; 4,14 (2H, q, J = 7 Hz) ; 4,85-6,15 (m, 5H) δ ppm ;

SM : $M^+$ = 168 (5) ; m/e : 122 (14), 94 (51), 80 (100), 79 (76), 67 (30), 55 (19), 41 (41), 29 (38).

Exemple 2

On a préparé une composition aromatisante de base de type fruité en mélangeant les ingrédients suivants (parties en poids) :

| | |
|---|---|
| Vanilline | 20 |
| Essence d'orange douce | 40 |
| Essence de citron | 10 |
| Acétate d'amyle | 20 |
| Acétate de butyle | 30 |
| Propionate d'éthyle | 30 |
| Butyrate d'éthyle | 50 |
| Valérate d'éthyle | 20 |
| Oenanthate d'éthyle | 40 |
| Alcool benzylique | 200 |
| Propylène-glycol | 540 |
| Total | 1000 |

A l'aide de la composition de base ci-dessus, on a préparé deux nouvelles compositions définies

respectivement comme composition de « contrôle » et composition « test », cette dernière étant obtenue en ajoutant de l'octa-cis-4,7-diène-1-oate d'éthyle comme suit :

| | Composition A (contrôle) | Composition B (test) |
|---|---|---|
| Composition de base | 100 | 100 |
| Octa-cis 4,7-diène-1-oate d'éthyle | — | 35 |
| Ethanol 95 % | 900 | 865 |
| Total | 1 000 | 1 000 |

Ces nouvelles compositions ont été soumises à une évaluation organoleptique dans du sirop acidulé (préparé en dissolvant 650 g de saccharose dans 1 000 ml d'eau contenant 10 ml d'une solution aqueuse à 50 % d'acide citrique), à raison de 100 g de composition pour 100 l de sirop acidulé. La composition « test » possédait une note caractéristique d'ananas, note qui était absente de la composition de « contrôle » de type « tutti-frutti » générique.

## Revendications

1. Un ester aliphatique polyinsaturé de formule

$$CH_2{=}CH{-}CH_2{-}\overset{4}{CH}{=}CH{-}CH_2{-}CH_2{-}\overset{O}{\overset{\|}{C}}{-}OR \qquad (I)$$

dans laquelle le symbole R représente un radical alkyle inférieur, linéaire ou ramifié, contenant 1 à 3 atomes de carbone et dans laquelle la double liaison à la position 4 possède la configuration cis.

2. Octa-cis 4,7-diénoate d'éthyle.

3. Utilisation d'un ester aliphatique polyinsaturé de formule (I), telle qu'indiquée à la revendication 1, en tant qu'ingrédient parfumant pour la préparation de parfums et produits parfumés, et en tant qu'agent aromatisant pour l'aromatisation d'aliments, de boissons, de préparations pharmaceutiques et du tabac.

4. Procédé pour la préparation d'un ester aliphatique polyinsaturé de formule (I), telle qu'indiquée à la revendication 1, caractérisé en ce qu'on

a) époxyde sélectivement le cycloocta-1,5-diène pour fournir le 1,2-époxy-cyclooct-5-ène,

b) oxyde le composé ainsi obtenu pour donner le 1-oxo-2-hydroxy-cyclooct-5-ène,

c) traite ledit céto-alcool avec du tétraacétate de plomb en milieu éthanolique pour fournir le 7-formyl-hept-cis-4-ène-1-oate d'éthyle,

d) réduit ce dernier à l'aide de borhydrure de sodium,

e) estérifie le 8-hydroxy-oct-cis 4-ène-1-oate d'éthyle résultant pour fournir le 8-acétoxy-oct-cis 4-ène-1-oate d'éthyle et

f) soumet ledit ester à pyrolyse pour fournir l'octa-cis 4,7-diène-1-oate d'éthyle, et, le cas échéant, transestérifie ledit ester.

## Claims

1. A polyunsaturated aliphatic ester of formula

$$CH_2{=}CH{-}CH_2{-}\overset{4}{CH}{=}CH{-}CH_2{-}CH_2{-}\overset{O}{\overset{\|}{C}}{-}OR \qquad (I)$$

wherein symbol R represents a lower, linear or branched alkyl radical having 1 to 3 carbon atoms and wherein the double bond in position 4 possesses the cis-configuration.

2. Ethyl octa-cis 4,7-dien-1-oate.

3. Utilization of a polyunsaturated aliphatic ester of formula (I) according to claim 1, as perfuming ingredient for the preparation of perfumes and perfumed products, and as flavouring ingredient for the aromatization of foodstuffs, beverages, pharmaceutical preparations and tobacco.

4. Process for the preparation of a polyunsaturated aliphatic ester of formula (I) according to claim 1, which comprises

a) selectively epoxydizing cycloocta-1,5-diene to give 1,2-epoxy-cyclooct-5-ene,

b) oxidizing the thus obtained compound to give 1-oxo-2-hydroxy-cyclooct-5-ene,

c) treating said keto-alcohol with lead tetraacetate in an ethanolic medium to give ethyl 7-formyl-hep-cis-4-en-1-oate,

d) reducing this latter by means of sodium borohydride,

e) esterifying the resulting ethyl-8-hydroxy-oct-cis 4-en-1-oate to yield ethyl 8-acetoxy-oct-cis 4-en-1-oate, and

f) subjecting said ester to pyrolysis to give ethyl octa-cis 4,7-dien-1-oate, and, when desired, transesterifying said ester.

**Ansprüche**

1. Ein mehrfach ungesättigter aliphatischer Ester der Formel

$$CH_2{=}CH{-}CH_2{-}\overset{4}{C}H{=}CH{-}CH_2{-}CH_2{-}\overset{O}{\overset{\|}{C}}{-}OR \qquad (I)$$

worin R eine geradkettige oder verzweigte niedrige Alkylgruppe mit 1 bis 3 Kohlenstoffatomen darstellt und worin die Doppelbindung in der 4-Stellung die cis-Konfiguration aufweist.

2. Ethyl-octa-cis-4,7-dien-1-oat.

3. Verwendung eines mehrfach ungesättigten aliphatischen Esters gemäß Anspruch 1, als Riechstoff zur Herstellung von Parfümen und parfümierten Produkten, und als Geschmacksstoff zur Aromatisierung von Lebensmitteln, Getränken, pharmazeutischen Präparaten und Tabak.

4. Verfahren zur Herstellung eines mehrfach ungesättigten aliphatischen Esters gemäß Anspruch 1, dadurch gekennzeichnet, daß man

a) Cycloocta-1,5-dien selektiv epoxidiert um 1,2-Epoxy-cyclooct-5-en zu erhalten,

b) das so erhaltene Produkt oxidiert um 1-Oxo-2-hydroxy-cyclooct-5-en zu erhalten,

c) den erhaltenen Keto-alkohol mit Bleitetraacetat in ethanolischem Milieu umsetzt um Ethyl-7-formyl-hept-cis-4-en-1-oat zu erhalten,

d) dieses mittels Natriumborhydrid reduziert,

e) das erhaltene Ethyl-8-hydroxy-oct-cis-4-en-1-oat verestert und Ethyl-8-acetoxy-oct-cis-4-en-1-oat erhält, und

f) den genannten Ester pyrolysiert um Ethyl-octa-cis-4,7-dien-1-oat zu erhalten und gewünschtenfalls diesen Ester umestert.